# EUROPEAN PATENT APPLICATION

(11) **EP 4 748 291 A1**
(43) Date of publication of application: **27.05.2026**
(21) Application number: 25213675.9
(22) Date of filing: 05.11.2025
(51) Int. Cl.: A61B 1/00, A61B 1/018

(54) **GUIDING ARRANGEMENT AND SURGICAL INSTRUMENT**

(30) Priority: 21.11.2024 DE 102024134351
(71) Applicant: Karl Storz SE & Co. KG, 78532 Tuttlingen (DE)
(72) Inventor: Juhanson, Timo, 78532 Tuttlingen (DE)

(57) **Abstract**

The invention relates to a guiding arrangement for receiving and guiding a tool of a surgical instrument, in particular of a single-use flexible endoscope, comprising a working shaft adaptor; a tubular working shaft connected to the working shaft adaptor and comprising a working channel extending beyond the working shaft adaptor; and a curved guiding element with a distal end and a proximal end, wherein the distal end is connected to the working shaft adaptor such that the curved guiding element guides the working channel along a curved trajectory to the proximal end. Furthermore, the invention relates to a corresponding surgical instrument, in particular a single-use flexible endoscope.

## Description

### FIELD OF THE INVENTION

The present invention relates to a guiding arrangement for receiving and guiding a tool of a surgical instrument. The present invention further relates to a surgical instrument, in particular a single-use flexible endoscope.

### BACKGROUND OF THE INVENTION

Flexible endoscopes offer a distinctive ability to access areas that are invisible to the naked eye, such as small cavities and viscera. Especially, single-use flexible endoscopes compared to re-usable endoscopes can reduce handling and cleaning efforts, which is becoming an increasingly important factor for surgical or medical procedures in clinics.

Furthermore, the miniaturization of flexible endoscopes enables enhanced maneuverability and accessibility to previously inaccessible areas of targets, such as the human body. It is therefore anticipated that there is potential for improving in patient outcomes and for reducing of trauma associated with interventional surgery procedures.

Accordingly, there is a growing trend towards single-use flexible endoscopes with a more compact structure and lower cost. A flexible endoscope comprises a bendable working shaft having at least one working channel extending along the axial direction of the flexible working shaft. The proximal portion of the working channel is attachable to an inlet port or manifold that is mountable on the surgical instrument. Owing to the configuration of the endoscope-handle, the working channel, as well as the loaded tools within, is arranged in the assembly state with a curvature in the housing of the surgical instrument or the handle. The curvature may exhibit a sharper or larger radius, which is contingent on the connecting angle of the mounted inlet port or manifold. In the ideal configuration with an appropriate curvature, the relevant portions of the working channel align respectively with the connecting counterparts, namely the axis of the inlet port connector and the axis of the working shaft.

To achieve the aforementioned benefits of single-use endoscopes, namely easy montage and lower cost without cleaning efforts, the endoscopes need to withstand stresses and forces from tools arranged in the working channel. Accordingly, the working channels tend to have a larger wall thickness and resulting diameter than necessary.

### SUMMARY OF THE INVENTION

In view of the above, an object of the present invention is to provide a novel and improved guiding arrangement for receiving and guiding surgical or medical tools, as well as a surgical instrument, particularly a single-use flexible endoscope, equipped with the novel guiding arrangement.

In accordance with the present invention, a guiding arrangement with the features of claim 1 and a surgical instrument with the features of claim 12 are provided. Advantageous or preferred features of the invention are recited in the dependent claims.

Accordingly, the present invention provides:
- Guiding arrangement for receiving and guiding a tool of a surgical instrument, in particular a single-use flexible endoscope, comprising a working shaft adaptor; a tubular working shaft connected to the working shaft adaptor and comprising a working channel extending beyond the working shaft adaptor; and a curved guiding element with a distal end and a proximal end, wherein the distal end is connected to the working shaft adaptor such that the curved guiding element guides the working channel along a curved trajectory to the proximal end.
- Surgical instrument, in particular a single-use flexible endoscope, comprising a housing; a guiding arrangement according to the invention; and an inlet port being mountable on the housing at an angle to the working shaft, wherein the working channel is guided by the guiding element to the inlet port such that a tool inserted in the inlet port is guided inside the working channel along the guiding element.

The underlying discovery upon which the present invention is based is that structurally connecting the working shaft and the working channel by using of a curved guiding element facilitates assembling and manufacturing process and further improves the structural stability of the working channel in use of the surgical instrument.

The concept upon which the present invention is based is therefore to configure such a working shaft adaptor with an extending portion in form of a curved guiding element to the inlet port, which provides support for the bending portion of the working channel as well as the loaded tools in the housing of the single-use flexible endoscope.

The guiding arrangement is accordingly configured for guiding the working channel all the way from the inlet port to the working shaft, preferred to the distal end of the working shaft. In this way, the working channel is provided with a durable support in its curved region, so that the working channel is capable of withstanding all internally encountered forces, especially for receiving and guiding a tool of a surgical instrument inserted in the working channel. Furthermore, the relatively uncomplicated configuration renders the guiding arrangement suitable for large-scale production and appropriate for application in single-use flexible endoscopes.

Advantageously, the working channel with its inserted tools, especially in case of a sharper bending radius, is protected by the guiding element from detachment from the inlet port or damaged by the tool. Accordingly, the present invention provides durable support in a flexible endoscope that does not result in a loss of maneuverability. In addition, the working channel supported by the guiding element provides enough resilience to resist fatigue or permanent deformation.

It is favorable to configure the guiding arrangement of the present invention such that the curved guiding element forms an integral or a separable part of the working shaft adaptor, thereby extra hinge supports can be advantageously implemented at at least one edge of the guiding element configured as an open channel in order to ensures supporting the curved portion of the loaded working channel. In this configuration, the guiding element is switched from an open to a closed state by engaging the hinge supports. Thus, this function-integrated configuration comprises thereby less components and ensures no damage or dislodging of the loaded working channel.

The majority components of the guiding arrangement, as well as of the surgical instrument, of the present invention may be produced by injection molding. The benefits of the manufacturing method and the material used in the injection molding process, such as suitability for bending and folding, can significantly reduce the efforts and difficulties associated with large-scale production.

The housing of the surgical instrument may be configured for being handled by means of a handgrip, which may be connected to or integrated in the housing. Alternatively or in addition, the housing may be configured for being handled by means of a surgical robot.

Furthermore, the conventionally multiple elements of a single-use flexible endoscope, such as adaptors, ports, and seals, according to the present invention can be integrated into a single-piece injection-molded work-piece, forming the guiding arrangement. Hence, this novel configuration offers a more cost-effective disposable flexible endoscope solution. With the inventive configuration, particularly the guiding arrangement, the manufacturing of the working shaft as well as the attached elements becomes easier and more resistant to faults.

Moreover, a guiding arrangement according to the invention allowing a single-use layout meets the requirement of providing surgical instruments and systems with lower efforts for cleaning and sterilization. Especially, the present invention enables an advantageous solution for sterile single-use parts for surgical instruments, such as single-use flexible endoscopes, wherein the working channel is firmly hold in this type of endoscope and will be disposed after the operation to obviate the necessity for subsequent dismantling and cleaning.

Advantageous designs and further embodiments result from the further dependent claims as well as from the description with reference to the figures in the drawing.

In an embodiment, the distal end of the guiding element is formed in one part with the working shaft adaptor, in particular in a form-locking manner. In this way, no further manufacturing is necessary. In order to provide support for the curved working channel, the guiding element is configured as an elongated portion of the working shaft adaptor. The guiding element is configured to be bent, advantageously as the working channel.

In another embodiment, the guiding element is configured to be engaged with the working shaft adaptor, in particular in a form-locking manner. This separable configuration allows for the injection-molded guiding element to be easily assembled with the working shaft by simply clipping into the guiding arrangement, particularly in the working shaft adaptor. This facilitates the mounting of the suitable guiding element in the matching housing of the endoscope.

In an embodiment, the guiding element and the working shaft adaptor are configured as a one-piece injection-molded work-piece. The function-integrated configuration of the working shaft adaptor has only two parts, ensuring no dislocation or dislodging compared to conventional designs. The compact one-piece construction of the guiding arrangement simplifies the mounting process of the flexible working shaft, which forms the single-use flexible endoscope.

In accordance with a preferred embodiment, the guiding element is configured as an open channel, in particular having a C-shaped cross section and/or with an opening on a side wall extending along a complete length of the guiding element. In this configuration, the working channel can be inserted either from a proximal or a distal end along the longitudinal axis into the guiding element, or alternatively pushed in the channel from the opening provided on the side wall. Due to the elasticity of the material, the opening can be expanded peripherally during the pushing action.

In a preferred embodiment, the side wall is configured to provide circumferential support for at least half the periphery of the working channel. In particular, the guiding element may be configured in such a way that two-thirds of the working channel is peripherally wrapped, thereby preventing dislodgement. The wall structured of the open channel can be one layer, multi-layer or multi-layer with braiding inside for additional support.

In another embodiment, at least one locking member is provided at at least one edge along the opening and configured to secure the working channel in position inside the guiding element. In addition to the open channel, the hinge-supports or locking members can be further provided to ensure the assembly of the working channel into the guiding element. Any discrepancies in form between the open channel and the working channel, which may arise during the assembly process, are rectified. The working channel is then deposited in the housing with the same curvature as the guiding element.

According to another embodiment, the locking member is configured to be folded over from an open state to engage with a corresponding notch provided on the opposite edge of the opening in a closed state. The presence of at least one notch at the edge facilitates a form-matched engagement with the corresponding locking member at the opposite edge. This configuration provides an additional assurance to prevent dislodgement.

In a preferred embodiment, the locking member has a base hinged to, preferably resiliently biased to, the edge. The locking member is through the base integral, particularly material-locking, with the guiding element at at least one edge. The biased material utilized in this configuration can ensures that the locking member withstand repeated folding without yielding, breaking or compromising its structural integrity. The advantageous properties of the applied material provide a guarantee on engagement after numerous folding cycles. In the inventive configuration, the loaded working channel and the guiding element can be assembled either in an open state or in a closed state.

In another embodiment, the locking member has a hook-shaped head configured to engage with a corresponding notch at the other edge of the opening. The hook-shaped head facilitates the form-locked engagement with the corresponding locking structure, e.g. notch, provided at the opposite edge. Furthermore, during the welding process, the material on the hook-shaped head can be provided as part of the welding material for material-locking engagement.

According to an embodiment, the locking member is formed in one piece with the guiding element, preferably by injection molding. Injection molding offers a range of benefits and advantages, e.g. three-dimensional forming and elasticity. Additionally, the production process allows for utilizing materials with favorable properties, e.g. elastic polymers, elastomers, or thermoplastic elastomers.

In accordance with another embodiment, the locking member is configured to be firmly bonded with the opposite edge in a closed state, preferably by welding. It is unnecessary to reopen the locking member after performing the single-use flexible endoscope, which makes this configuration the optimal choice for this particular type of endoscope.

Concerning a single-use flexible endoscope, once the locking members are welded on the guiding element to enclose the working channel, there is no necessity for further opening by force. Consequently, the design affords surgeons the opportunity to select the optimal surgical approach for each patient.

According to an embodiment of a surgical instrument, the inlet port is configured as a straight inlet port, in particular formed as an injection-molded work piece, such that a tool inserted straight in the inlet port is guided along the curved trajectory of the guiding element into the tubular working shaft. Especially, the present invention allows for the use or mounting of a straight inlet port on a single-use flexible endoscope, which can be manufactured on a large scale, e.g. by using injection molding. This results in a reduction in the cost per unit of the single-use flexible endoscope is achieved. Furthermore, the straight inlet port ensures that the working channel has a straight path for inserting a tool to the inlet port. Consequently, the medical or surgical tool is less likely to be stuck on the edge of the working channel.

In another embodiment of a surgical instrument, a proximal connecting end of the working channel is stretchable over a connector of the inlet port. The straight inlet port, which is utilized for coupling to the working channel, can be mounted on the housing of the endoscope with minimal effort, the entire assembly is completed, and the functionality of the single-use flexible endoscope is achieved. A similar design can be applied to the manifold. The configuration utilizing stretching-over provides a more firmly bond over the glue-in method.

Furthermore, the stretched-over with an aligned mounting angle allows for a more straightforward mounting process that does not damage the working channel or the loaded tools within it.

In a preferred embodiment of a surgical instrument, the guiding arrangement is configured according to the invention and at least one locking member is configured to secure a connection of the working channel and the inlet port. In this manner, the present invention offers a novel and improved surgical instrument, in particular a single-use flexible endoscope, featured by the inventive guiding arrangement. The innovative guiding element serves as the principal supporting portion, thereby allowing for the optimization of the configuration of the endoscope housing and mountable components thereon. The bending part, which is arranged in the housing of the endoscope, can thus be designed with an appropriate length and corresponding curvature.

The above embodiments can be combined with each other as desired, if useful. Further possible embodiments, further configurations and implementations of the invention also include combinations, not explicitly mentioned, of features of the invention described herein with respect to the embodiments. In particular, the skilled person will thereby also add individual aspects as improvements or additions to the respective basic form of the present invention.

### BRIEF DESCRIPTION OF THE DRAWINGS

Aspects and advantages of the present invention will be better understood with reference to the following figures accompanied by the detailed description, which emphasize upon clearly illustrating the principles of the present disclosure. In the different figures, the same elements are indicated with the same reference numerals, in which:
- Fig. 1: is a schematic perspective view of a guiding arrangement in an open state arranged within a housing of a surgical instrument;
- Fig. 2: is an enlarged schematic perspective view of a connector of a straight inlet port;
- Fig. 3: is a schematic perspective view of a loaded guiding arrangement in a closed state disposed in a housing of a surgical instrument;
- Fig. 4: is a schematic perspective view of a connector of a straight inlet port;
- Fig. 5: is a schematic perspective view of a surgical instrument.

The accompanying drawings are included to provide a further understanding of the present invention and are incorporated in and constitute a part of this specification. The drawings illustrate particular embodiments of the invention and together with the description serve to explain the principles of the invention. Other embodiments of the invention and many of the attendant advantages of the invention will be readily appreciated as they become better understood with reference to the following detailed description.

It will be appreciated that common and/or well understood elements that may be useful or necessary in a commercially feasible embodiment are not necessarily depicted in order to facilitate a more abstracted view of the embodiments. The elements of the drawings are not necessarily illustrated to scale relative to each other. It will further be appreciated that certain actions and/or steps in an embodiment of a method may be described or depicted in a particular order of occurrences while those skilled in the art will understand that such specificity with respect to sequence is not actually required. It will also be understood that the terms and expressions used in the present specification have the ordinary meaning as is accorded to such terms and expressions with respect to their corresponding respective areas of inquiry and study, except where specific meanings have otherwise been set forth herein.

### DETAILED DESCRIPTION OF EMBODIMENTS

The detailed description includes specific details for providing a thorough understanding of the present invention. However, it will be apparent to those skilled in the art that the various described elements or features may be arranged in various combinations and configurations without departing from the scope of the present invention.

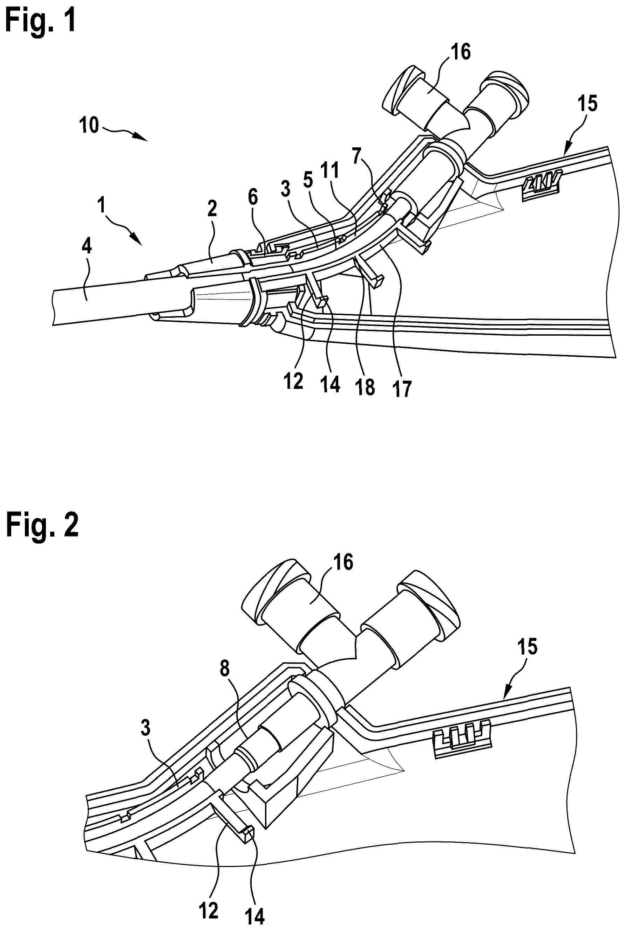

Fig. 1 depicts a schematic perspective view of a guiding arrangement 1 in an open state arranged within a housing 15 of a surgical instrument 10.

The surgical instrument 10 can represent a single-use flexible endoscope. The illustrated portion of the guiding arrangement 1 is locally arranged and supported within the housing 15. For illustrative purpose, the top part of the housing 15 has been removed.

The guiding arrangement 1 for receiving and guiding tools of the surgical instrument 10 comprises principally a working shaft adaptor 2, a curved guiding element 3, and a tubular working shaft 4 with a working channel 5.

A noticeable attribute of the flexible surgical instrument 10 is the bendable tubular working shaft 4. The working channel 5 with the surgical or medical tools is loadable within the working shaft 4 and extends along the longitude axis thereof.

The loaded working shaft 4 is assembled with the working shaft adaptor 2 by inserting the proximal portion thereof through a front opening of the working shaft adaptor 2. The working shaft adaptor 2 with the loaded working shaft 4 is then detachably attached to the housing 15 of the surgical instrument 10. In an assembly state, the working shaft adaptor 2 is fluid-proof coupled to the housing 15, for example by means of a form fit or form lock connection.

A proximal connecting end 8 of the working channel 5 extends along a longitudinal axis of the working shaft adaptor 2 to a connector 9 of an inlet port 16. The inlet port 16 is preferably configured as a straight inlet port that is detachably mounted on the housing 15 of the surgical instrument 10, wherein the proximal connecting end 8 of the working channel 5 is preferably stretched over the connector 9.

As illustrated in Fig. 1, the working channel 5, along with the integrated tools, is disposed in the housing 15 with a curvature. To support the bending portion of the working channel 5 and to prevent any potential disengagement or damage of the loaded working channel 5, a curved guiding element 3 is positioned within the housing 15.

The guiding element 3 has a distal end 6 and a proximal end 7, with the distal end being connectable to the working shaft adaptor 2. The curved and bendable configuration of the guiding element 3 allows it to guide the working channel 5 along a curved trajectory to the proximal end 7 that is attachable to the inlet port 16.

The guiding element 3 can be configured as a standalone separable component with the distal end 6 that is configured to be form-locking engaged in the working shaft adaptor 2. This can be achieved, for instance, by sliding the distal end 6 into the working shaft adaptor 2 and snapping into the position with pins or holders securing it in place. The locking mechanism at the distal end 6 ensures a secure connection, preferred, a fluid-proof engagement is provided by using additive rubber material. The guiding element 3 of this configuration can be readily and rapidly dismounted and remounted in order to provide an appropriate dimension for the housing 15.

Preferably, the working shaft adaptor 2 and the guiding element 3 are configured as a single-piece injection-molded work-piece, especially injection molded with an elastic material. This configuration permits the efficient manufacture and assembly of the surgical instrument 10. During the mounting process, the working shaft 4 is initially coupled to the working shaft adaptor 2 with the extended guiding element 3. Subsequently, the entire assembly is guided through the front opening into the housing 15 of the endoscope.

The guiding element 3 is configured as an open channel and it is preferred that the cross section perpendicular to the longitudinal axis exhibits a C-shaped profile. An opening 11 is provided on a side wall of the guiding element 3. The working channel 5 guided in the housing 15 can be pressed from the opening 11 into the open channel. During this assembly process, the opening 11 may be widened radially and/or the working channel may temporarily deform due to elasticity of the applied material. Alternatively, the working channel 5 can be inserted into the guiding element 3 along the longitudinal axis. The working channel 5 extends from the proximal connecting end 8 that is attached to the connector 9, to a distal end of the working shaft 4.

The configuration of the side wall of the guiding element 3 ensures that, in an assembled state, the side wall provides circumferential support for at least half, and preferably more than half, e. g. two-thirds, of the periphery of the working channel 5, in particular of the outer perimeter.

At least one locking member 12 is arranged at at least one edge 17 along the opening 11 hinged to, preferably resiliently biased to, the edge 17. It is not obligatory for all of the locking members 12 to be located at only one of the edges 17; alternatively or in addition, the locking members 12 may be positioned in a staggered configuration on either edge 17 of the opening 11. The provision of the locking members 12 is to secure the working channel 5 in position inside the guiding element 3.

Fig. 2 depicts an enlarged schematic perspective view of the connector 9 of the inlet port 16, which is mounted on the housing 15 of the surgical instrument 10.

The inlet port 16 is preferably configured as a straight inlet port. This configuration facilitates the tool insertion procedure without incident or error, in particular with reduced stresses and/or forces from tools inserted.

The working channel 5 is adjustably arranged within the housing 15 with an appropriate curvature of the disposed guiding element 3 guiding the working channel 5 to the loaded working channel 5. The proximal connecting end 8 can be aligned with the connecting terminal according to this configuration and arrangement.

Fig. 2 illustrates the straight inlet port 16, which exhibits a T-shaped configuration. In the mounting state, one terminal for coupling to the working channel 5 is situated within the housing 15, while the remaining two terminals are intended for inserting tools, e.g. for fluid communication with a fluid supply and/or suction system. In another embodiment, the inlet port 16 can be replaced by a manifold that is in use for connection and communication with other inevitable systems of the surgical instrument 10. The manifold provides the same functions for connecting the working channel 5 and the tools within.

As mentioned, the inlet port 16 can be produced as a single-piece injection-molded component. The pervasive adoption of injection-molded work-pieces for the single/use flexible endoscope has the potential to result in a notable reduction in the overall cost of the instrument.

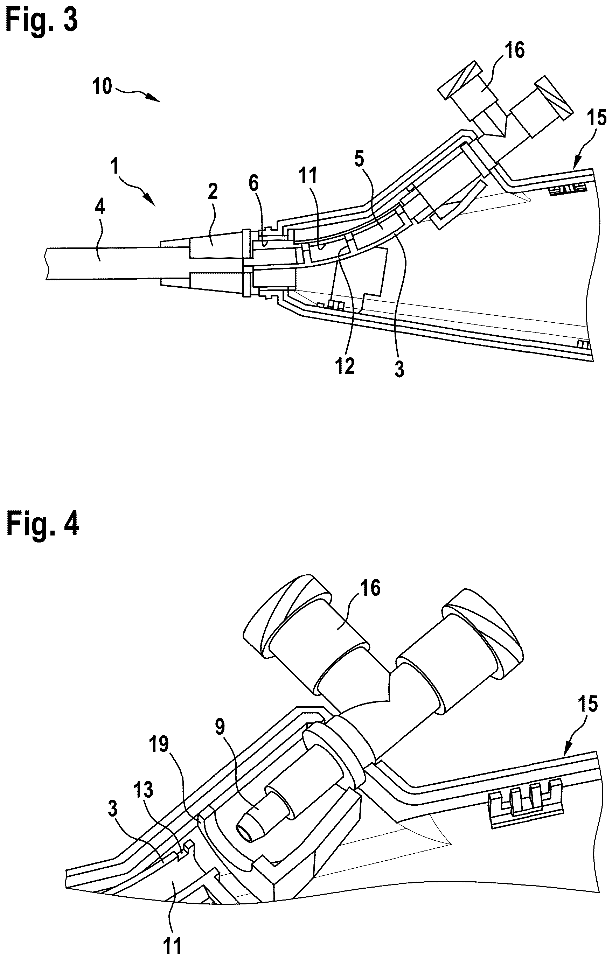

Fig. 3 is a schematic perspective view of a loaded guiding arrangement 1 in a closed state disposed in the housing 15 of the surgical instrument 10.

In order to from an open state achieve a closed state, the locking members 12 are configured to be folded over from the open state to engage with the opposite edge 17 of the opening 11 in a closed state. In the exemplary embodiment of Fig. 3, a corresponding notch 13 is provided on the opposite edge 17 of the opening 11.

It should be noted that the number of locking members 12 provided is not limited, only as an illustrative example Fig. 3 shows three locking members 12. Generally, such locking members 12 can be arranged either at the same edge 17 or at both edges 17 of the opening 11, for example alternating on the first edge and on the second edge.

In an advantageous embodiment as shown in Fig. 3, the locking members 12 are equipped with a hook-shaped head 14 that is designed to be engaged in the notch 13 on the opposite edge 17 in the closed state. The locking mechanism on both elements ensures a firmly coupled connection.

In the case of a single-use flexible endoscope, there is no necessity to transform again the locking member 12 from the engagement to the disengagement after implementation. Consequently, with or without a head 14, the locking member 12 can be configured to be firmly bonded with the opposite edge 17, such as in the notch 13, in a closed state, preferably by welding.

As illustrated in Fig. 3, the working channel 5 is secured in the guiding element 3 with the locking member 12 closed. An angle between the connector 9 and the longitude axis of the working shaft 4 results in the curvature of the working channel 5 and the supporting guiding element 3 being positioned in the housing 15 defining this curvature. The characteristics of the utilized material, such as resilience, can ensure a firmly locking connection under force resulting from distortion and loading tools.

The locking member 12 can be formed as an integral part of the guiding element 3, wherein the locking member 12 and the guiding element 3 are configured as one piece, preferably by injection molding. The locking member 12 has a base 18 that is hinged to, preferably resiliently biased to the edge 17, for example in the manner of an integral hinge or film hinge. The application of the elastic material in the injection molding process ensures that the configuration during the assembly process without dislodging.

Fig. 4 depicts a schematic perspective view of a connector 9 of the straight inlet port 16.

As enlarged illustrated in Fig. 4, the guiding element 3 guides the working channel 5, not shown in Fig. 4, to the connector. The working channel 5 extends along and beyond the longitude dimension of the guiding element 3 and connects with the connector 9. For example, the proximal connecting end 8 of the working channel 5 is stretched over the connector 9, which therefore has a conical tip. A support fastener 19, that may be integrally configured on the inside surface of the housing 15, is capable of supporting the proximal portion of the working channel 5. This structure helps further to prevent the dislodgment of the working channel 5.

Once the proximal connecting end 8 has been firmly coupled to the connector 9, the locking members 12 can be folded over and welded closed, thus securing the working channel 5 in position.

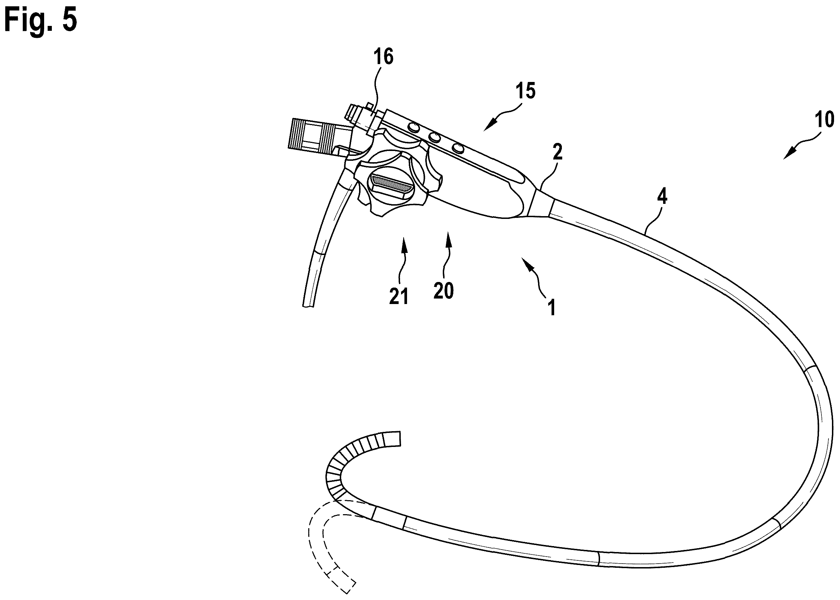

Fig. 5 depicts a schematic perspective view of the surgical instrument 10.

The surgical instrument 10 is configured as a flexible endoscope, on which the guiding arrangement 1 is implemented. The flexible working shaft 4 is attached to the working shaft adapter 2, which in turn is connected to the housing 15 of the flexible endoscope. The working channel 5 (not visible in Fig. 5) that is loaded in the working shaft 4 is extended into the housing 15 via the working shaft adapter 2 and up to the inlet port 16, where the working channel 5 is stretched over the connector 9 of the inlet port 16.

The housing 15 of the surgical instrument 10 is configured for being handled by means of a handgrip 20 integrated in the housing and its flexible distal end can be steered by means of steering elements 21, here for example steering wheels. In other embodiments, the housing and steering may be configured for being handled by means of a surgical robot.

Although specific embodiments of the invention are illustrated and described herein, it will be appreciated by those of ordinary skill in the art that a variety of alternative and/or equivalent implementations exist. It should be appreciated that the exemplary embodiment or exemplary embodiments are examples only and are not intended to limit the scope, applicability, or configuration in any way. Rather, the foregoing summary and detailed description will provide those skilled in the art with a convenient road map for implementing at least one exemplary embodiment, it being understood that various changes may be made in the function and arrangement of elements described in an exemplary embodiment without departing from the scope as set forth in the appended claims and their legal equivalents. Generally, this application is intended to cover any adaptations or variations of the specific embodiments discussed herein.

It will also be appreciated that in this document the terms "comprise", "comprising", "include", "including", "contain", "containing", "have", "having", and any variations thereof, are intended to be understood in an inclusive i.e. non-exclusive sense, such that the process, method, device, instrument, apparatus or system described herein is not limited to those features or parts or elements or steps recited but may include other elements, features, parts or steps not expressly listed or inherent to such process, method, article, or apparatus. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects.

### LIST OF REFERENCE SIGNS

- 1: guiding arrangement
- 2: working shaft adaptor
- 3: guiding element
- 4: working shaft
- 5: working channel
- 6: distal end
- 7: proximal end
- 8: proximal connecting end
- 9: connector
- 10: surgical instrument
- 11: opening
- 12: locking member
- 13: notch
- 14: head
- 15: housing
- 16: inlet port
- 17: edge
- 18: base
- 19: fastener
- 20: handgrip
- 21: steering elements

## Claims

1. Guiding arrangement (1) for receiving and guiding a tool of a surgical instrument (10), in particular of a single-use flexible endoscope, comprising:
a working shaft adaptor (2);
a tubular working shaft (4) connected to the working shaft adaptor (2) and comprising a working channel (5) extending beyond the working shaft adaptor (2); and
a curved guiding element (3) with a distal end (6) and a proximal end (7), wherein the distal end (6) is connected to the working shaft adaptor (2) such that the curved guiding element (3) guides the working channel (5) along a curved trajectory to the proximal end (7).

2. Guiding arrangement (1) according to claim 1,
wherein the distal end (6) of the guiding element (3) is formed in one part with or configured to be engaged with the working shaft adaptor (2), in particular in a form-locking manner.

3. Guiding arrangement (1) according to claim 1,
wherein the guiding element (3) and the working shaft adaptor (2) are configured as a one-piece injection-molded work-piece.

4. Guiding arrangement (1) according to any one of the preceding claims, wherein the guiding element (3) is configured as an open channel, in particular having a C-shaped cross section and/or with an opening (11) on a side wall extending along a complete length of the guiding element (3).

5. Guiding arrangement (1) according to any one of the preceding claims, wherein the side wall is configured to provide circumferential support for at least half the periphery of the working channel (5).

6. Guiding arrangement (1) according to any one of the preceding claims, wherein at least one locking member (12) is provided at at least one edge (17) along the opening (11) and configured to secure the working channel (5) in position inside the guiding element (3).

7. Guiding arrangement (1) according to claim 6,
wherein the locking member (12) is configured to be folded over from an open state to engage with a corresponding notch (13) provided on the opposite edge (17) of the opening (11) in a closed state.

8. Guiding arrangement (1) according to claim 6 or 7,
wherein the locking member (12) has a base (18) hinged to, preferably resiliently biased to, the edge (17).

9. Guiding arrangement (1) according to one of the claims 6 to 8,
wherein the locking member (12) has a hook-shaped head (14) configured to engage with a corresponding notch (13) at the other edge (17) of the opening (11).

10. Guiding arrangement (1) according to one of the claims 6 to 9,
wherein the locking member (12) is formed in one piece with the guiding element (3), preferably by injection molding.

11. Guiding arrangement (1) according to one of the claims 6 to 10,
wherein the locking member (12) is configured to be firmly bonded with the opposite edge (17) in a closed state, preferably by welding.

12. Surgical instrument (10), in particular a single-use flexible endoscope, comprising:
a housing (15);
a guiding arrangement (1) according to any one of the preceding claims; and
an inlet port (16) being mountable on the housing (15) at an angle to the working shaft (4),
wherein the working channel (5) is guided by the guiding element (3) to the inlet port (16) such that a tool inserted in the inlet port (16) is guided inside the working channel (5) along the guiding element (3).

13. Surgical instrument (10) according to claim 12,
wherein the inlet port (16) is configured as a straight inlet port, in particular formed as an injection-molded work piece, such that a tool inserted straight in the inlet port is guided along the curved trajectory of the guiding element (3) into the tubular working shaft (4).

14. Surgical instrument (10) according to claim 12 or 13,
wherein a proximal connecting end (8) of the working channel (5) is stretchable over a connector (9) of the inlet port (16).

15. Surgical instrument (10) according to one of claims 12 to 14,
wherein the guiding arrangement (1) is configured according to one of claims 6 to 11 and at least one locking member (12) is configured to secure a connection of the working channel (5) and the inlet port (16).
